# EUROPEAN PATENT APPLICATION

(11) **EP 4 079 243 A1**
(43) Date of publication of application: **26.10.2022**
(21) Application number: 21170180.0
(22) Date of filing: 23.04.2021
(51) Int. Cl.: A61B 18/12, A61B 18/14, A61B 18/00

(54) **SENSING FOR A CATHETER**

(71) Applicant: Koninklijke Philips N.V., 5656 AG Eindhoven (NL)
(72) Inventor: Faingersh-Klebanov, Anna, 5656 AE Eindhoven (NL); IBRAGIMOV, Zalman, 5656 AE Eindhoven (NL); SCHWARTZ, Yitzhack, 5656 AE Eindhoven (NL); SHLAIN, Adar Shlain, 5656 AE Eindhoven (NL)
(74) Representative: Philips Intellectual Property & Standards

(57) **Abstract**

A mechanism for providing a trigger signal that is usable to control the collection of electrode calibration data from a catheter. The trigger signal is responsive to a predictive indicator that indicates a likelihood that the catheter is in contact with the anatomical structure, and in particular examples, that the catheter is completely immersed in blood. The predictive indicator is generated using a ratio between two measurements that change responsive to touching the anatomical structure.

## Description

### TECHNICAL FIELD

The present disclosure relates generally to sensing for a catheter, such as contact sensing for the catheter.

### BACKGROUND

Catheters, such as ablation catheters, are becoming increasingly used in the medical treatment of patients. For example, atrial fibrillation (AF) is an abnormal heart rhythm characterized by rapid and irregular beating of the atria, and may be associated with heart palpitations, fainting, lightheadedness, shortness of breath, or chest pain. The disease is associated with an increased risk of heart failure, dementia, and stroke. AF may be caused by electrical pulses generated by secondary pacers at the ostium of the pulmonary veins. Accordingly, one way of treating AF is by pulmonary vein isolation, which can include ablating the inner wall of the left atrium to form lesions that isolate the ostium of the pulmonary veins from the rest of the left atrium.

Ablation can be performed in various ways, including radiofrequency (RF) ablation, ultrasonic ablation, pulsed field ablation and cryoablation. RF ablation is a conventional ablation procedure that involves powering an electrode of a catheter to create contiguous, transmural lesions using heat energy.

To provide effective ablation, it would be advantageous to properly position the catheter against the anatomical structure or feature to be ablated. It would be particularly advantageous if a contact between the catheter and the anatomical structure could be identified and, preferably, measured. Of course, other types of catheter can also benefit from contact detection with an anatomical structure (e.g. for insertion of devices, such as pacemakers/stents or for the penetration of tissue, e.g. to perform biopsies or a transsceptal puncture).

Several catheters have been developed in order to sense the contact force level. These catheters tend to rely upon force sensors including complex mechanical elements (springs for example) at the tip of the catheter to detect whether or not contact is made.

Other known approaches, such as that suggested by WO 2019/215721 A1, propose to measure impedance e.g. across a gap between electrodes in order to estimate a contact force applied by a catheter to tissue of a patient. Yet another approach is suggested by US 2012/283715 A1, which proposes to correlate a capacitive measurement value to determine a contact force between a catheter and tissue (i.e. an anatomical structure).

Thus, in some known examples, measurements at one or more electrodes of a catheter are used to predict whether the catheter is in contact with the anatomical structure.

### SUMMARY

Aspects of the present disclosure provide devices, systems, and methods for generating electrode calibration data for improving the determination of a contact between a catheter and an anatomical structure (e.g. an organ, blood vessel or the like).

It has previously been explained how the determination of a contact between a catheter and an anatomical structure can use measurements obtained from one or more electrodes of the catheter. To improve the performance of this prediction, it would be advantageous to obtain electrode calibration data, which can be used to calibrate or "zero" future measurements taken by the catheter. This electrode calibration data should be obtained whilst (e.g. all the electrodes of) the catheter is not in contact with the anatomical structure, e.g. (for cardiac use-case scenarios) when (e.g. all of the electrodes of) the catheter are entirely (and solely) in contact with the blood.

There is proposed an approach for controlling when to obtain the electrode calibration data. In particular, there is proposed an approach for generating a trigger signal for controlling when to obtain the electrode calibration data.

In particular, there is proposed a processor circuit for generating and outputting a trigger signal for controlling the collection of electrode calibration data from a catheter comprising two or more electrodes for performing ablation therapy on an anatomical structure.

The processor circuit is configured to communicate with the ablation therapy catheter, the processor circuit comprising: an input interface configured to obtain: a first measurement, from a first set of one or more electrodes of the catheter, responsive to a change in contact and/or distance between the first set of two or more electrodes and the anatomical structure; and a second measurement, from a second, different set of one or more electrodes of the catheter, responsive to a change in contact and/or distance between the second set of two or more electrodes and the anatomical structure, and a data processor communicatively coupled to the input interface and configured to: determine a ratio between the first and second measurements; and process the determined ratio to generate a predictive indicator that indicates a likelihood that the ablation therapy catheter is in contact with the anatomical structure; and generate and output a trigger signal that changes responsive to the predictive indicator.

There is proposed an approach for controlling when to obtain electrode calibration data for calibrating or "zeroing" measurements obtained from electrodes of a catheter.

The present invention proposes to determine a ratio between a first and second measurement obtained from a respective first and second set of electrodes of the catheter. This ratio is used to generate a predictive indicator that indicates a likelihood that the ablation therapy catheter is in contact with the anatomical structure. In particular examples, the predictive indicator may indicate a likelihood that the electrodes of the catheter is in contact with only blood (i.e. not in contact with the anatomical structure, i.e. not in contact with tissue).

The predictive indicator is then used to control the trigger signal, which is configured for controlling the collection of electrode calibration data (i.e. controlling the timing of collecting electrode calibration data).

Using a ratio between two measurements to control when to obtain the electrode calibration data provides a mechanism for accurate, automated obtaining of the electrode calibration data, as the value of the ratio is independent of blood conductivity (e.g. unlike measurements obtained directly from the electrodes), but still (at least partially) responsive to the catheter touching the anatomical structure - allowing for accurate identification of when the catheter is in the blood.

Preferably, the catheter is an ablation catheter and the first set of one or more electrodes of the catheter comprise one or more electrodes used to apply an ablation treatment to the anatomical structure.

In some embodiments, the first measurement comprises an impedance measurement between a first set of two electrodes of the catheter. Optionally, the second measurement comprises an impedance measurement between a second set of two electrodes of the catheter, wherein the second set is different to the first set.

The inventors have recognized that a ratio between impedance measurements provides a particularly suitable parameter for triggering the obtaining of calibration data. However, other measurements obtainable from a set of one or more electrodes of a catheter are also usable.

In some examples, the data processor is configured to process the determined ratio by processing the determined ratio and the first measurement to generate the predictive indicator. In other words, the step of processing the determined ratio may comprising using a function that receives, as input, at least the determined ratio and the first measurement and provides, as output, the predictive indicator.

In some examples, the input interface is configured to receive a physiological signal, the physiological signal responsive to a respiratory and/or cardiac movement of the patient; and the data processor is configured to process the determined ratio by processing the determined ratio and the physiological signal to generate the predictive indicator. The magnitude of the measurements obtained from electrodes of a catheter are affected by respiratory and/or cardiac activity. By taking these measurements into account, the accuracy of the trigger signal can be improved.

In some examples, the data processor is configured to process the determined ratio by comparing the determined ratio to one or more first predetermined values and/or ranges to generate a predictive indicator that predicts whether or not the ablation therapy catheter is in contact with the anatomical structure.

In preferable examples, the data processor is configured process the determined ratio by processing the determined ratio using a probability function to calculate a probability that the ablation therapy catheter is in contact with the anatomical structure; and generate the trigger signal responsive to the calculated probability. Generating a probability provides an adaptable and flexible approach for defining the trigger signal.

The probability function may define a predetermined mapping between possible values for the determined ratio and possible values for the probability. In particular, the mapping may be a bell or Gaussian distribution about a predetermined value for the ratio. The predetermined value may be specific to the catheter, and can be calculated or predetermined in advance (e.g. through testing or the like).

In some examples, the data processor is configured to generate the trigger signal to carry the calculated probability. Thus, the trigger signal may comprise the calculated probability, so that control over the obtaining of the calibration data is directly responsive to the calculated probability.

There is also proposed a processing arrangement comprising any herein descried processor circuit; and processing circuitry configured to: receive the trigger signal output by the processor circuit; and selectively obtain electrode calibration data, from one or more of the electrodes of the catheter, responsive to the trigger signal.

The processing arrangement may be configured, wherein: the processor circuit is the processor circuit configured to generate the trigger signal to carry the calculated probability; and the processing circuitry is configured to obtain the electrode calibration data in response to the calculated probability, contained in the trigger signal, exceeding a predetermined probability threshold.

There is also proposed a (computer-implemented) method for generating and outputting a trigger signal for controlling the collection of electrode calibration data using an ablation therapy catheter comprising two or more electrodes for performing ablation therapy to an anatomical structure.

The method comprises: obtaining a first measurement, from a first set of one or more electrodes of the catheter, responsive to a change in contact and/or distance between the first set of two or more electrodes and the anatomical structure; obtaining a second measurement, from a second set of one or more electrodes of the catheter, responsive to a change in contact and/or distance between the second set of two or more electrodes and the anatomical structure; determining a ratio between the first and second measurements; processing the determined ratio to generate a predictive indicator that indicates a likelihood that the ablation therapy catheter is in contact with the anatomical structure; and generating and outputting a trigger signal that changes responsive to the predictive indicator.

There is also proposed a computer program product comprising code which, when executed by a processor circuit, causes the processor circuit to perform the steps of any herein descried method. There is also proposed a non-transitory computer-readable medium or data carrier comprising or carrying the computer program product.

The present disclosure also proposes a computer program (product) comprising instructions which, when the program is executed by a computer or processing system, cause the computer or processing system to carry out (the steps of) any herein described method.

The skilled person would be readily capable of adapting any herein described method to reflect embodiments of herein described apparatus, systems and/or processor circuits, and vice versa. A similar understanding would be made by the skilled person with respect to a computer program (product).

The processor circuit can be comprised in an apparatus for determining a state of contact between a catheter, such as for example an RF catheter, and a tissue of a subject before, during and after ablation procedure. In embodiments such apparatus may thus comprise a computer (mobile (phone, tablet laptop) or stationary (desktop or workstation or console of a medical device such as dielectric imaging or mapping system or electro-anatomical mapping system or other electrophysiological medical system). In embodiments the processor circuit or device may be comprised in a console of a medical device such medical device may be a dielectric or electromagnetic imaging or mapping system. The catheter thus also may be an electrophysiology catheter for measuring electrophysiological parameters. Such catheters include EP catheters as known in the art used for electro-anatomical mapping etc.

Additional aspects, features, and advantages of the present disclosure will become apparent from the following detailed description.

### BRIEF DESCRIPTION OF THE DRAWINGS

Illustrative embodiments of the present disclosure will be described with reference to the accompanying schematic drawings, of which:
Fig. 1 illustrates an ablation system;
Fig. 2 is a flowchart illustrating a method;
Fig. 3 illustrates impedance measurements and a ratio between the impedance measurements for different blood conductivities;
Fig. 4 illustrates impedance measurements and a ratio between the impedance measurements at different points during an investigation process;
Fig. 5 illustrates a probability function;
Fig. 6 illustrates different measurements obtainable from electrodes on a catheter;
Fig. 7 is a flowchart illustrating another method;
Fig. 8 illustrates additional components for an ablation system; and
Fig. 9 illustrates a processor circuit.

### DETAILED DESCRIPTION

For the purposes of promoting an understanding of the principles of the present disclosure, reference will now be made to the embodiments illustrated in the drawings, and specific language will be used to describe the same. It is nevertheless understood that no limitation to the scope of the disclosure is intended. Any alterations and further modifications to the described devices, systems, and methods, and any further application of the principles of the present disclosure are fully contemplated and included within the present disclosure as would normally occur to one skilled in the art to which the disclosure relates.

The present disclosure proposes a mechanism for providing a trigger signal that is usable to control the collection of electrode calibration data from a catheter. The trigger signal is responsive to a predictive indicator that indicates a likelihood that the catheter is in contact with the anatomical structure, and in particular examples, that the catheter is completely immersed in blood. The predictive indicator is generated using a ratio between two measurements that change responsive to touching the anatomical structure.

Embodiments are based on the realization that a ratio between two measurements responsive to touching the anatomical structure is independent of blood conductivity. This means that a more accurate identification of when the catheter is in the blood pool.

The underlying concept may be used to improve the calibration of obtaining measurements from a catheter.

The following disclosure is described in the context of an ablation system, in which the catheter is an ablation therapy catheter for performing on ablation on an anatomical structure such as for example a cardiac wall. However, the proposed concepts are suitable for use with any catheter (having two or more electrodes) for insertion into one or more cavities of an anatomical structure, e.g. for the purpose of treatment and/or surgery. By way of example, the catheter may comprise an ablation therapy catheter (as herein described), a device insertion catheter for inserting a device into a subject/patient (e.g. a pacemaker or stent), a biopsy catheter (e.g. for obtaining one or more biopsy samples), a puncturing catheter (e.g. for puncturing through part of an anatomical structure, e.g. for performing a transsceptal puncture) and so on.

The one or more cavities may include any suitable cavity, passage or vessel of a patent/subject which is bound by an anatomical stricture (e.g. formed of tissue), and may be filled with blood, air or any other suitable fluid or semi-fluid. A catheter not in contact with the anatomical structure (or tissue) may be considered to be fully in contact with the fluid or semi-fluid that fills the cavity (e.g. blood or the like).

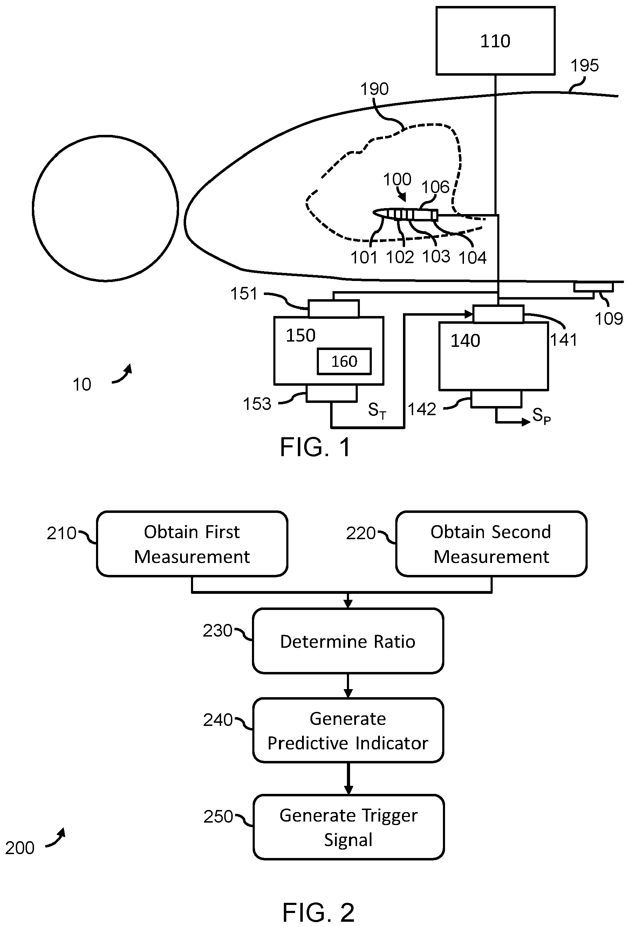

Fig. 1 provides a graphical view of an ablation system 10, comprising an ablation therapy catheter 100, one example of a suitable catheter for use with the present invention. The ablation therapy catheter is illustrated as being in operation within an anatomical structure 190 (e.g. a heart) of a patient/subject 195. Other examples of suitable anatomical structures will be described later.

The ablation therapy catheter 100 comprises two or more electrodes 101-104 mounted upon a flexible elongate member 106. In some embodiments, the catheter comprises 4 electrodes (as illustrated), namely: a first electrode 101, a second electrode 102, a third electrode 103 and a fourth electrode 104. However, the catheter can include other numbers of electrodes, including 2, 4, 6, 14, 16, 20, 24, 30, 60, or any other suitable number of electrodes. The flexible elongate member 106 enables the ablation therapy catheter 100 to be positioned at desired locations within the anatomical structure 190.

One or more of the electrodes 101-104 of the ablation therapy catheter 100 are designated "ablation electrodes", which can controllably ablate the anatomical structure 190. Ablation can be carried out by controlling an alternating current ("ablation current") supplied to the ablation electrode(s) 101-104 in order to appropriately control an output (e.g. electrical field intensity output by or heat of) the ablation electrode(s).

In one example of ablation, radiofrequency ablation (RFA), heat is generated by supplying a medium frequency alternating current (e.g. in the range of (in the range of 350-500 kHz) to each ablation electrode. The heat generated by the ablation electrode(s) ablates/damages surrounding tissue (i.e. tissue of the anatomical structure 190).

However, ablation electrodes may be used to perform other forms of ablation, such as pulsed field ablation (PFA), sometimes called electroporation. Pulsed field ablation is a non-thermal ablation procedure that uses high voltage bursts which are delivered in close proximity to tissue, which results in nanoscale pores (electroporation) being induced in cell membrane, which can result in cell death (i.e. ablation of tissue).

Ablation electrodes may be controlled to perform a combination of different ablation procedures during an overall ablation procedure (e.g. a combination of RFA and PFA).

Any number of the electrodes 101-104 may act as an ablation electrode, so that there may be only 1 ablation electrode or a plurality of ablation electrodes. Preferably, the ablation therapy catheter 100 is configured so that at least the most distally positioned electrode 101 (a "first electrode") can be controlled to selectively ablate nearby tissue, e.g. generate or not generate heat, i.e. is an ablation electrode.

Ablation occurs during RFA when sufficient current is supplied to one or more ablation electrodes that results in the ablation electrodes heating to a degree that would result in suitable ablation damage (for intended medical purposes) if brought into contact with tissue. Ablation occurs during PFA when sufficient current/voltage is supplied to the one or more ablation electrodes that results in electroporation in desired tissue (if the ablation electrode(s) were brought sufficiently proximate to the desired tissue).

The operation of the ablation electrode(s) 101-104 of the ablation therapy catheter 100 can be controlled by an ablation controller 110 of the ablation system 10. The ablation controller 110 may, for example, control the magnitude of a current supplied to the ablation electrode(s) 101-104. In particular, the ablation controller 110 may be electrically connected (e.g. by one or more wires positioned within the flexible elongate member 106) to the ablation electrode(s) 101-104, to thereby control a current supplied thereto.

In particular, the ablation controller 110 may comprise an electrical signal generator configured to control the electrodes of the catheter to emit and/or acquire or sense electrical signals, including voltages and currents.

The magnitude of the current supplied to the ablation electrode(s) 101-104 may be automatically controlled (e.g. to meet some predetermined ablation scheme) or manually controlled via one or more of the mechanisms described hereinafter.

Temperature based feedback of ablation current or power during RFA is often used.

The ablation controller 110 may be configured to receive a location of the catheter 100 (e.g. from a mapping system), determine if the location matches a predetermined location where ablation is to be performed, and performing ablation if the location matches the predetermined location. A suitable process for automatic ablation is described by the U.S. Patent Application having publication number No. 2018/310987, titled "Systems and Processes for Map-Guided Automatic Cardiac Ablation".

The ablation electrode(s) may be irrigated or non-irrigated. Irrigated electrodes may permit the delivery of fluid (e.g. sterile fluid such as saline) to the irrigations to cool the electrode interface. This can allow higher power delivery for a longer duration without the formation of coagulum. The catheter may be appropriately adapted to permit the delivery of water (e.g. comprise a fluid line or the like). The delivery of fluid may be controlled by the ablation controller.

Electrodes 101-104 not used for ablation may be labelled "non-ablation electrodes".

These electrodes may be used, optionally together with the ablation electrodes, to map the anatomical structure and/or determine a relative location of the catheter with respect to the anatomical structure. This process is, however, entirely optional.

Approaches for using electrodes to map anatomical structure (body volumes) and visualize the locations of catheters within the map can be based on electrical, magnetic or combined tracking principles as known in the art. Examples, can be found in, for example, U.S. Patent No. 10,278,616, titled "Systems and Methods for Tracking an Intrabody Catheter," filed May 12, 2015, and U.S. Patent No. 5,983,126, titled "Catheter Location System and Method," filed August 1, 1997, the entireties of which are hereby incorporated by reference. Thus, the electrodes may be in electrical communication with a mapping system (not shown) for mapping the anatomical structure and/or identifying a relative location of the catheter with respect to the anatomical structure.

In the illustrated example, the first electrode 101 is an ablation electrode. The second 102, third 103 and fourth 104 electrodes are non-ablation electrodes. However, other configurations will be apparent to the skilled person (e.g. using different total numbers of electrodes, ablation electrodes and/or non-ablation electrodes).

The skilled person will appreciate that appropriate placement of the ablation therapy catheter 100, and appropriate control of the magnitude of the current provided to the ablation electrode(s) thereof, facilitate ablation of (elements of features of) the anatomical structure on/in which the ablation therapy catheter operates.

Further details regarding suitable catheters and assemblies can be found in, for example, U.S. Patent No. 6,002,955, titled "Stabilized Electrophysiology Catheter and Method for Use," the entirety of which is hereby incorporated by reference, or the previously mentioned U.S. Patent Application having a publication number of No. 2018/310987.

Fig. 1 further illustrates processing circuitry 140 which is configured to predict or detect a touch between the catheter and the anatomical structure 190.

The processing circuitry 140 comprises an input interface 141, which is configured to obtain, from two or more electrodes 101-104 "contact detection electrodes", at least one electrical response of each electrode of the ablation therapy catheter to an electric field or electric fields in the anatomical structure. This may be performed by electrically connecting the processing circuitry 140 to the contact detection electrodes, e.g. via wires disposed in the flexible elongate member), to facilitate detection of electric fields. Some or all of these wires may be shared with the ablation controller 110.

Accordingly, the processing circuitry 140 may comprise an electrical signal measurer configured to detect electrical signals at the (contact detection) electrodes of the catheter, i.e. to determine an electrical response of electrodes of the catheter. Electrical signals may comprise or consist of voltages and/or currents in complex form (e.g. amplitude and phase of alternating signals) or not. From those impedances may be determined or calculated, but this need not be the case.

Although illustrated as a separate element, the processing circuitry 140 may, in some examples, be incorporated into the ablation controller 110. In other examples, the processing circuitry 140 may be incorporated into some other component of the system 10.

An electrical response of an electrode may be a measurement of a particular electrical parameter (e.g. voltage or capacitance) associated with that electrode to an electric field present in the anatomical structure. In particular, an electrical response may be a measurement of a response of a particular electrical parameter (e.g. voltage, current or capacitance) of that electrode to an electric field generated by an electrode of the catheter.

Electrical responses may be processed to obtain other forms of measurement not directly derivable from a single electrode, e.g. an impedance measurement between two electrodes.

Thus, measurements may be obtained from a plurality of electrodes (e.g. voltage measurements, current measurements, capacitance measurements, resistance measurements, impedance measurements and so on).

Different electric fields may vary in magnitude at different (near-unique) frequencies (i.e. have different frequencies). This enables a processing circuitry 140 to distinguish between the effects of different electric fields upon an electrode (i.e. as different fields can be identified by virtue of the near-unique frequency), using appropriate filters to attribute different frequencies of an overall response to a different electric field.

In other words, the response of an electrode to a particular electric field can be individually identified using frequency-based filtering (as different electric fields may be associated with different sources). The skilled person would be readily capable of using appropriate digital/analog filters to attribute different frequency components of an overall response of an electrode to a particular electric field (associated with a particular frequency or frequencies).

These electric fields may be electric fields generated by the electrodes(s) 101-104 themselves, which electric fields may be in the region of 10-100 kHz, e.g. between 10kHz and 25kHz. However, other ranges may also be used. Each electrode may generate an electric field of a different frequency, so that the electrical response of an electrode to a field generated by a particular electrode can be identified. The electric fields may, for example, be controllably generated by the processing circuitry and/or the ablation circuit and/or some other controller in electrical communication with the electrodes (e.g. a controller for a mapping system for identifying a relative location of the catheter).

In the context of the present disclosure, an electrical response may be any suitable electrical measurement of an electrical characteristic that is responsive to a change in a magnitude of an electric field in the vicinity of the electrode. In particular, the electrical response may include any suitable electrical measurement that responds to a change in a dielectric property (e.g. dielectric constant) in material between the contact detection electrode and the source of an electric field (to which the contact detection electrode responds). Thus, an electrical response may include a voltage response, a current response, a capacitance response and so on.

As a first example, a voltage response may be a voltage detected by an electrode at a particular frequencies (e.g. with different frequency being associated with different electric fields, e.g. different electrodes). As indicated hereinbefore, such voltage response may be a complex voltage with amplitude and phase.

Thus, an overall voltage response (of an electrode) may be formed of one or more different voltage values, e.g. (V₁, V₂, V₃) each associated with a different electric field, e.g. (E₁, E₂, E₃). Each voltage value represents a different voltage response.

A voltage value may be a (average) voltage difference between the relevant electrode and a reference electrode or a ground/earth (at the particular frequency for the particular electric field). Each voltage value may be an average (e.g. RMS) voltage detected at an electrode for a different electric field (e.g. by filtering based on predetermined frequencies).

In the context of the present disclosure, a voltage response of an i-th electrode to an electric field generated by a j-th electrode may refer to a (average) voltage difference between the i-th electrode and a ground/reference (e.g. a voltage at a reference electrode) at a frequency of a current supplied to the j-th electrode.

As a second example, an overall capacitance response (of an electrode) may indicate a capacitance between a particular electrode and a known originating source of the electric field. Thus, an overall capacitance response may comprise two or more capacitance values, e.g. (C₁, C₂, C₃) each associated with a different electric field, e.g. (E₁, E₂, E₃). Each capacitance value represents a different capacitance response.

In some embodiments, the processing circuitry 140 (or other component such as a mapping system) may be configured to define or control an electric field emitted by an electrode. For example, the processing circuitry (or other component) may control a current supplied to an electrode. If the electrode is an ablation electrode, this current may be superimposed over the current supplied for ablation. The processing circuitry 140 may, in some examples, therefore comprise an electrical signal generator configured to control the electrodes of the catheter to emit electrical signals.

Each electrode may be controlled (by the processing circuitry 140 or a separate electric field controller) to emit an electric field of a different frequency. This can be performed by supplying an alternating current of the relevant frequency to each electrode (e.g. via wires disposed in the flexible elongate member 106). The frequencies emitted by the electrodes on the catheter are preferably in the region of 20-100 kHz (to avoid unintentional ablation of the anatomical structure, whilst being suited for contact (force) detection).

This approach results in a processing circuitry 140 being able to identify, in a response (e.g. voltage response) of an electrode, the effect of different electric fields generated by the different electrodes. Thus, the processing circuitry can determine the (electrical) response of an electrode to an electric field generated by another electrode.

The processing circuitry 140 is configured to process one or more electrical responses of one or more contact detection electrodes to determine/predict whether or not the catheter 100 makes contact with (tissue of) the anatomical structure. This information is used to generate the predictive signal S_{P} responsive to the prediction of whether or not the ablation therapy catheter is in contact with the anatomical structure.

This prediction may be binary (e.g. a prediction of whether or not the catheter is in contact with the anatomical structure), categorical (e.g. a prediction of a category of touch, such as "No Touch", "Touch" and "High Touch" or "Pressure Applied") and/or continuous (e.g. a touch value/measurement, whose magnitude depends upon a predicted intensity/force of a touch). The prediction may comprise a touch probability, which is a probability indicating a likelihood that the catheter touches the anatomical structure. Methods for generating such predictions are well known.

Optionally, this may be performed by predicting a contact force between the catheter and the anatomical structure (which indicates whether or not contact is made). Methods of predicting a contact force will be known to the skilled person, for example, WO 2019/215721 A1 discloses a method of predicting a contact force from impedance measurements.

Generally, without wishing to be bound by theory, a magnitude of a measured electric field increases when the contact force increases. This is because the magnitude of the measured electric field is at least partly dependent upon a geometry (as well as the magnitude of the electric field into which the electrode is placed). Thus, monitoring an electrical response that is responsive to a change in electric field amplitude facilitates monitoring of a change in contact force (indicating a contact has occurred or to predict a magnitude of a contact force). The skilled person would be readily capable of correlating an electric response of an electrode to a change in contact force based on this teaching.

Fig. 1 further illustrates a reference electrode 109, which may monitor/provide a reference voltage level (e.g. for determining a voltage response of a particular electrode). The reference electrode may an external electrode that provides a reference voltage level. The reference electrode may, for example, be positioned on a leg of the subject (e.g. a "right leg patch"). Other suitable positions would be apparent to the skilled person, e.g. the lower back or the pelvis. The reference voltage may be ground.

To improve the performance of the prediction of whether or not the catheter is in contact with the anatomical structure, the processing circuitry 140 may be configured to obtain electrode calibration data. The electrode calibration data is usable to calibrate measurements taken from the catheter 101, e.g. in a process commonly known as zeroing or baseline determination. For example, the electrode calibration data may comprise a baseline or "zero" measurement, being a measurement obtained when the catheter is not in contact with the anatomical structure (e.g., for a heart, so that all electrodes are entirely surrounded by blood - for a respiratory system, so that all electrodes are entirely surrounded by gas or the like). Future measurements may be compared to the baseline measurement to improve the identification of contact (or no contact) between the electrode(s) and tissue.

Preferably, the electrode calibration data is obtained by the processing circuitry responsive to some trigger signal S_{T}. The trigger signal may provide information usable for indicating when the catheter is considered to not be in contact with the anatomical structure, e.g. when the ablation electrodes are completely in the blood pool (when positioned in a cardiac system).

Historically, the trigger signal S_{T} is a signal responsive to a user input, so that user is able to control when the calibration or zeroing of the processing circuitry occurs. This requires attention of a user and is error prone.

In the illustrated example, the electrode calibration data is obtained by the processing circuitry 140. However, in other examples, the electrode calibration data may be obtained by other processing circuit (i.e. separate from the processing circuitry 140) that is communicatively coupled to the catheter. The electrode calibration data may then be separately passed to the processing circuitry 140).

Those elements of Fig. 1 that are not specific to ablation may be incorporated into other catheter systems (e.g. employing other types of catheter) previously described. For instance, the ablation therapy catheter 100 may be replaced by another type of catheter, and the ablation control may be omitted entirely. Where appropriate, the term "ablation system" and "ablation therapy catheter" may therefore be replaced by "catheter system" and "catheter" respectively, where appropriate.

The present invention proposes a mechanism for improving the provision of the trigger signal, and in particular, for providing an automated trigger signal and a further automated approach for performing zeroing or other calibration steps.

The inventive concept aims at overcoming the disadvantages of manual control of the trigger signal. In particular, the invention aims to reduce the error of manual zeroing, to make it adaptable to changing environment and to eliminate the need for manual zeroing, e.g. when user engagement for zeroing is not possible such as using this information for imaging.

Fig. 1 illustrates a processor circuit 150 according to an embodiment. The processor circuit 150 is configured to generate the trigger signal S_{T}, which is used (by the processing circuitry 140) to control when to obtain the electrode calibration data for performing zeroing or other calibration steps.

The processor circuit 150 comprises an input interface 151 that obtains measurements from two or more electrodes 101-104 of the catheter. The input interface is therefore communicatively coupled with the two or more electrodes.

The processor circuit 150 also comprises a data processor 160 communicatively coupled to the input interface 151. The data processor 160 processes the obtained measurements to generate the trigger signal S_{T}.

The trigger signal S_{T} may be output by the processor circuit at an output interface 152. The trigger signal S_{T} can then be passed to the processing circuitry 140 (e.g. via the input interface 141 of the processing circuitry 140) or other processing circuitry (if present) for controlling when to obtain the electrode calibration data.

Fig. 2 is a flow-chart illustrating a (computer-implemented) method 200 performed by the processor circuit 150 according to an embodiment. Reference to both Figs. 1 and 2 will be made for the following description.

The method 200 comprises a step 210 of obtaining a first measurement, from a first set of one or more electrodes of the catheter. The first measurement is responsive to a change in contact and/or distance between the first set of two or more electrodes and the anatomical structure.

The method 200 also comprises a step 220 of obtaining a second measurement, from a second, different set of one or more electrodes of the catheter. The second measurement is also responsive to a change in contact and/or distance between the second set of two or more electrodes and the anatomical structure.

The first and/or second measurement may be a voltage measurement, an impedance measurement, a capacitance measurement, a resistive measurement and so on. Some measurements, such as an impedance measurement, may be calculated from two or more electrical responses of the electrodes (e.g. a voltage response of a first electrode and a voltage response of a second electrode). Other measurements, such as a voltage measurement, may be equivalent to an electric response, for instance a voltage measurement may be equal to a voltage response of a particular electrode.

Steps 210 and 220 may be performed by the input interface 151.

Preferably, the first (or second) impedance is obtained from at least the ablation electrode of the catheter, if the catheter 100 is an ablation catheter.

The method 200 also comprises a step 230 of determining a ratio between the first and second measurements. This may, for instance, comprise dividing the first measurement by the second measurement or vice versa.

The method 200 also comprises a step 240 of processing the determined ratio to generate a predictive indicator that indicates a likelihood that the ablation therapy catheter is in contact with the anatomical structure. For a use-case scenario in cardiac structures such as the heart, the predictive indicator thereby indicates a likelihood that the catheter is entirely/solely in the blood pool, and not in contact with any anatomical structure.

The method 200 also comprises a step 250 of generating and outputting a trigger signal S_{T} that changes responsive to the predictive indicator. The trigger signal S_{T} may, for instance, carry (encoded) information about the predictive indicator, e.g. the predictive indicator itself. In other examples, the trigger signal S_{T} may contain a further processed predictive indicator - e.g. a (binary) indication as to whether electrode calibration data should be obtained or not.

The present invention recognizes that measurements that are responsive to change in contact/distance between the electrode(s) and the anatomical structure are also likely to vary with blood conductivity. In particular, impedance measurements have been identified as exhibiting this property. However, other forms of measurements (e.g. voltage measurements) have also been identified as exhibiting this property.

The present disclosure appreciates that monitoring a single measurement (or sum/average/product of multiple measurements) will be insufficient to accurately identify whether the catheter is not in contact with the anatomical catheter for the purpose of triggering the obtaining of electrode calibration data.

By using a ratio between two different measurements (obtained from two different sets of one or more electrodes), the impact of blood conductivity on the measurements is taken into account and mitigated. The ratio is also responsive to the catheter coming into contact with the anatomical structure (e.g. as part of the catheter will come into contact before other parts).

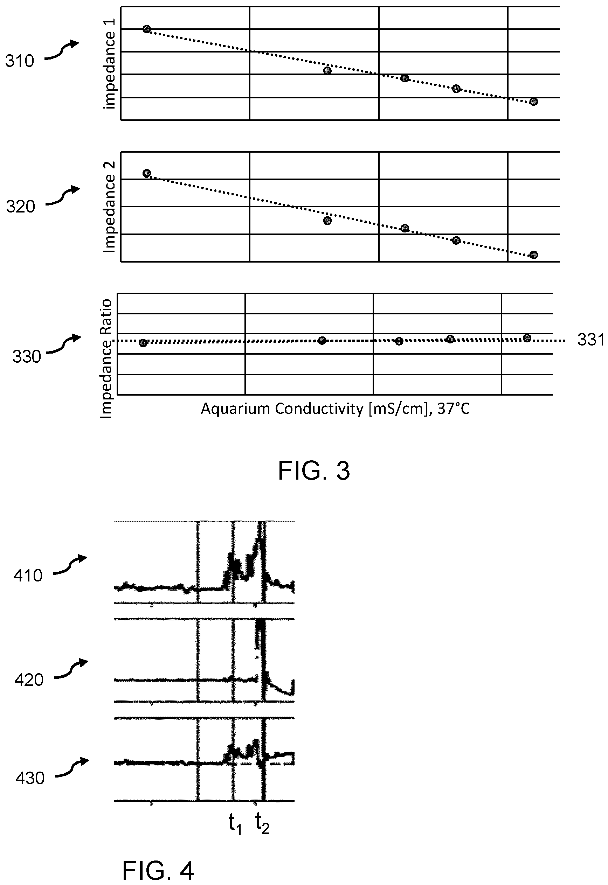

Fig. 3 illustrates the impact of blood conductivity on exemplary impedance measurements (being a measurement of impedance between two electrodes of a catheter). These impedance measurements were obtained using aquarium water (mimicking blood) having different conductivities, thereby providing synthetic blood conductivities.

A first waveform 310 shows an impedance measurement between a first set of electrodes for different synthetic blood conductivities ("Impedance 1"). A second waveform 320 shows an impedance between a second (different) set of electrodes for the same blood conductivities ("Impedance 2"). A third waveform 330 shows a ratio between these two impedance measurements ("Impedance Ratio").

Fig. 3 clearly illustrates that whilst the first and second impedance measurements decrease with increased blood conductivity, their ratio does not (significantly) change. Thus, the ratio is independent of blood conductivity, and (when immersed in blood) has a predetermined or determinable value 331. The value of the ratio when the catheter is immersed solely in blood (i.e. not in contact with an anatomical structure) may be specific to the catheter, and can be calculated or predetermined in advance (e.g. through testing or the like).

Fig. 4 illustrates the impact of the catheter touching the anatomical structure and the effect on the first impedance measurement, the second impedance measurement and the ratio between the first and second impedance measurements.

A first waveform 410 shows the first impedance measurement. A second waveform 420 shows the second impedance measurement and a third waveform 430 shows the third impedance measurement. These are all illustrated over a period of time before a touch between the catheter and the anatomical structure, and after a touch between the catheter and the anatomical structure (at a first time point t₁), as well as after a "high touch" (at a second time point t₂), where a high touch may indicate pressure being applied by the catheter to the anatomical structure. Immediately after the time point t₂ the catheter is removed from touching the anatomical structure.

It can also be seen that the ratio changes responsive to a touch (i.e. between time points t₁ and t₂), to differ from the predetermined value. Thus, the ratio acts as a good indicator of whether or not the catheter is in contact with the anatomical structure (and therefore whether electrode calibration data should be obtained), whilst being independent of blood conductivity.

Referring back to Fig. 2, the predictive indicator generated in step 240 may comprise a probability (e.g. a value on a scale of 0 to 1, 0 to 10, 1 to 10, 0 to 100 or 1 to 100). Thus, step 240 may comprise processing the determined ratio by processing the determined ratio using a probability function to calculate a probability responsive to a likelihood that the ablation therapy catheter is in contact with the anatomical structure. In some instances, the probability is a probability that the catheter is solely within the blood pool, P_{BP}.

Similarly, step 250 may comprise generating the trigger signal responsive to the calculated probability. For instance, step 250 may comprise generating a trigger signal that carries the calculated probability or generating a trigger signal that is responsive to the calculated probability.

In these examples, step 240 may comprise applying a probability function to the calculated ratio to generate the probability P_{BP}. The probability function may, for instance, directly map values of the ratio to a value of a probability, e.g. following a bell or normal distribution centered around the a/predetermined value for the particular catheter. If, for instance, the ratio is equal to the predetermined value, then the probability value may be equal to 1. Values for the ratio either side of this predetermined value may correspond to increasingly smaller probabilities.

The sigma or standard deviation of the bell distribution/curve may, for example, be between 0.05 and 0.1, e.g. 0.05.

Experimentally, the range of standard deviations between different types of catheter was: 0.01-0.04, so it would be preferred if the standard deviation of this bell distribution is greater than 0.04. Moreover, it was discovered that the standard deviation between ratios of different catheters was > 0.1, so it would be preferred for the standard deviation of this bell curve to be less than this value. A standard deviation that lies within the range of 0.05 and 0.1 thereby provides a suitable and appropriate value for accurate calculation of a probability.

The probability function may be specific to the particular catheter.

In some examples, the predetermined value may be dynamically changed over time, e.g. during a medical procedure making use of the catheter.

In one example, the predetermined value may be dynamically changed in response to a value for the first and/or second impedance falling below a previously recorded value for the first and/or second impedance respectively and the calculated ratio being within a predetermined range of the (current) predetermined value. The predetermined range may, for instance, be a range equal to the predetermined value ± the standard deviation of the bell distribution/curve. In other examples, the predetermined range may be predefined.

This embodiment recognizes that there is an expectation for the first/second impedance measurement to be at their lowest when positioned in the blood pool. Thus, if this value falls below some previously recorded (lowest) value, then this may indicate that a more accurate predetermined value is available.

The previously recorded value for the first and/or second impedance measurement may be a lowest recorded value for the first and/or second impedance measurement or a value of the first and/or second impedance measurement at a last time that calibration data was obtained (e.g. at a last time that a calculated probability exceeded some predetermined value).

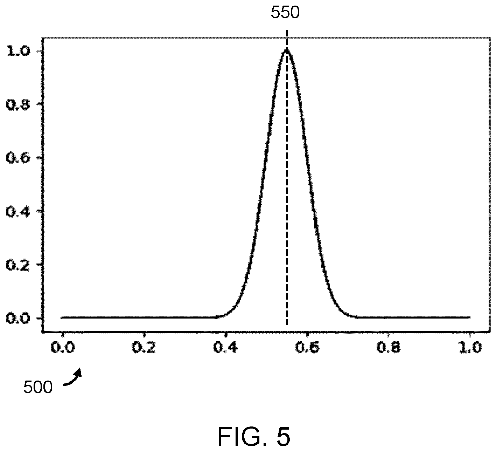

Fig. 5 provides a schematic illustration of the probability function 500, which maps a ratio (on the x-axis) to a probability (on the y-axis). The probability function is centered around the predetermined value 550 (where the probability is equal to 1, although this is not essential), and falls away either side thereof, e.g. to form a bell curve.

In Fig. 5, the probability indicated is a (numeric) probability that the ablation catheter is not in contact with the anatomical structure, e.g. is only in contact with the blood pool.

In other examples, the predictive indicator may be a categorical or binary predictive indicator that predicts whether or not the ablation therapy catheter is in contact with the anatomical structure. For instance, step 240 may process the determined ratio by comparing the determined ratio to one or more first predetermined values and/or ranges to generate a predictive indicator that predicts whether or not the ablation therapy catheter is in contact with the anatomical structure.

The trigger signal may carry the predictive indicator, e.g. to the processing circuitry, for controlling when to obtain the electrode calibration data.

To further improve the accuracy of the trigger signal, the predictive indicator may be responsive to the ratio between the first and second measurement and the first measurement (independently of the ratio). Thus, step 240 may comprise receiving, as separate inputs, the ratio and the first measurement and providing, as output the predictive indicator (e.g. a probability).

By way of example, consider a scenario where the predictive indicator is a probability ranging from 0 to 1, where 0 indicates a prediction that the catheter is in contact with the anatomical structure and 1 indicates a prediction that the catheter is not in contact with the anatomical structure (values in between indicating a likelihood in between these two extremes). In this scenario, the probability may be set to 0 if the first measurement indicates that the catheter is in contact with the anatomical structure (e.g. if the value of the first measurement exceeds some predetermined measurement threshold). If the first measurement indicates that the catheter is not in contact with the anatomical structure (e.g. does not breach the predetermined measurement threshold), then the probability may be calculated using the probability function.

In some embodiments, the predictive indicator may be responsive to the ratio between the first and second measurements and the predictive signal Sp.

Thus, the method 200 may comprise a step of obtaining the predictive signal (e.g. from the processing circuitry), where the predictive signal S_{P} is responsive to the prediction of whether or not the ablation therapy catheter is in contact with the anatomical structure. Step 240 may be appropriately configured to generate the predictive indicator based on the calculated ratio and the predictive signal.

For instance, consider a scenario where the predictive indicator is a probability ranging from 0 to 1, where 0 indicates a prediction that the catheter is in contact with the anatomical structure and 1 indicates a prediction that the catheter is not in contact with the anatomical structure. In this scenario, the probability may be set to 0 if the predictive signal S_{P} indicates that the ablation therapy catheter is in contact with the anatomical structure.

In this scenario, the probability may be calculated by processing the ratio using the probability function if the predictive signal indicates that the ablation therapy catheter is not in contact with the anatomical structure.

As another example for this scenario, if the predictive signal carries a touch probability, the probability may be calculated by combining the output of the probability function (that processes the ratio as previously described) and the touch probability, e.g. multiplying the two together.

These embodiments (generating based on predictive signal S_{P} and generating based on first measurement) improve the accuracy of the trigger signal, and in particular, the predictive indicator.

In some embodiments, the predictive indicator may be further responsive to electrogram signals, e.g. intracardiac electrogram (IEGM) raw signals, obtained via one or more electrodes of the catheter. For instance, the predictive indicator may be configured to indicate a high probability that the catheter is touching the anatomical structure in response to detecting electrogram activity in the electrogram signals - as this indicates that the electrode(s) is/are touching tissue, which leads to improved electrical signals conduction.

Thus, the method 200 may comprise a step of obtaining electrogram signals (via the user interface) from the one or more electrodes of the catheter. An electrogram signal is a signal responsive to changes in heart electrical activity. The step 240 may comprise generating the predictive indicator based on the ratio and the electrogram signals.

It has previously been described how the electrical response(s) of one or more electrodes of the catheter can be used to map the anatomical structure (e.g. body volumes) and visualize the locations of catheters within the map. This could be performed by the processing circuitry or by other processing equipment. This information can be used to predict a distance between the catheter and the anatomical structure. This is commonly called a "distance to mesh" measurement, or simply "distance to mesh".

The predictive indicator may be further responsive to the distance to mesh measurement. For example, where the predictive indicator is a probability, the indicated likelihood that the catheter is in the blood pool may decrease responsive to the distance to mesh decreasing.

Thus, the method 200 may comprise a step of obtaining distance to mesh information (e.g. from the processing circuitry). The step 240 may comprise generating the predictive indicator based on the ratio and the electrogram signals.

The magnitude of the electrical responses (and measurements obtained therefrom) are affected by respiratory and/or cardiac activity. Moreover, the effect of respiratory and/or cardiac activity magnitude of the electrical responses increases when the catheter makes contact with the anatomical structure.

In some embodiments, the method 200 is configured to receive a physiological signal (e.g. at the input interface), the physiological signal responsive to a respiratory and/or cardiac movement of the patient.

The step 240 may be configured to process the determined ratio by processing the determined ratio and the physiological signal to generate the predictive indicator. In other words, step 240 may comprise generating the predictive indicator based on the physiological signal and/or the ratio.

Any combination of the previously proposed embodiments may be performed, for instance, to generate the predictive indicator based on a physiological signal (or signals), the ratio and electrogram signals.

The above examples disclose approaches in which a probability is generated, however, the skilled person would readily appreciate how they could be adapted for generating other forms of predictive indicator, e.g. a binary indicator or a categorical indicator.

All embodiments makes use of measurements obtained from a first and second set of one or more electrodes of the catheter. Preferably, the first and second sets of electrodes are different. Even more preferably, the first and second sets comprises adjacent electrodes (i.e. electrodes adjacent upon the catheter), as this will improve the specificity of the generation of the trigger signal.

Fig. 5 illustrates some measurements, obtainable from the electrodes of the catheter, that could be used for different embodiments of the present disclosure.

A first example measurement Z_{1,2}, being an impedance measurement, between the first electrode 101 and second electrode 102 is illustrated. The impedance can be calculated using a voltage response of each electrode, and in particular, a voltage value of each electrode associated with an electric field generated by one of the electrodes, as previously described (e.g. with reference to equation 1). A current between the two electrodes can also be calculated during the calculation of the impedance.

A second example measurement Z_{2,3} being another impedance measurement, between the second electrode 102 and the third electrode 103 is illustrated. This impedance can be generated in a similar manner to the first example measurement.

The first example measurement can act, for the purposes of the present invention, as the first measurement and the second example measurement can act, for the purposes of the present invention, as the second measurement. In this scenario, the first set of electrodes therefore comprises the first and second electrodes, and the second set of electrodes comprises the second and third electrodes.

However, other measurements (or combinations of measurements) could also be used, and could include a voltage induced in one electrode by another electrode, an impedance between two electrodes, a capacitance between two electrodes and so on. In particular, any suitable measurement responsive to changes in a dielectric property could be used.

For instance, a third example measurement, being a voltage measurement V_{2,3}, is the voltage response of the second electrode 102 to a fixed-frequency alternating current supplied to the third electrode 103, i.e. an electric field generated by the third electrode 103.

A fourth example of a suitable measurement is a voltage response Vi,i of the first electrode 101 to a fixed-frequency alternating current supplied to/by the first electrode 101, i.e. an electric field generated by the first electrode 101. Another example is an impedance measurement of an impedance Z_{3,4} between the third electrode 103 and the fourth electrode 104.

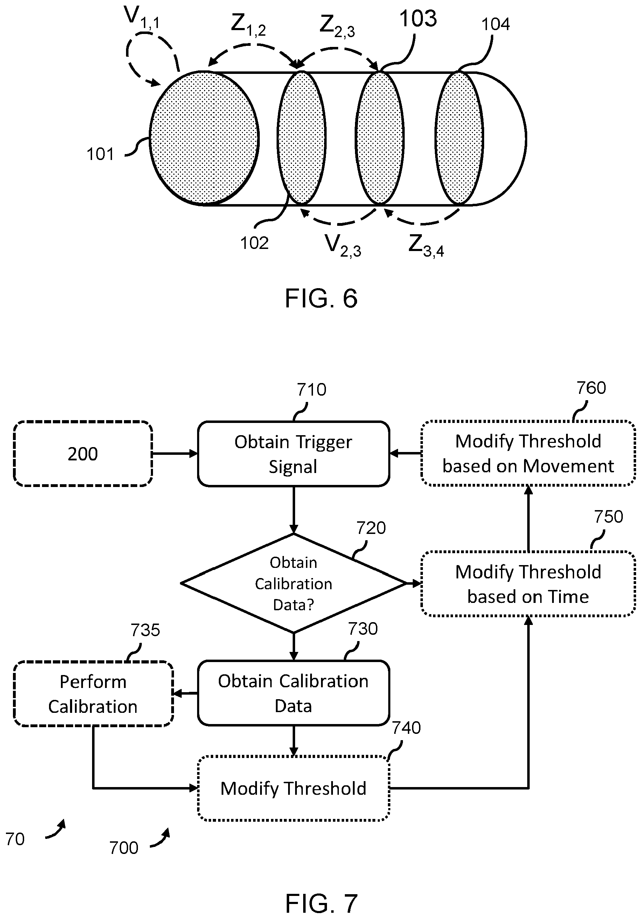

Fig. 7 illustrates a method according to a further embodiment of the invention. The method 700 may be performed by the processing circuity 140, or other processing circuitry configured to generate/obtain calibration data. Fig. 7 also illustrates an overall method 70 according to another embodiment of the invention, which comprises the method 700.

The method 700 comprises a step 710 of obtaining a trigger signal, e.g. from the processor circuit 150. Thus, the method 700 may obtain the trigger signal output by the method 200 (which forms part of the method 70).

For the purposes of the following description, it is assumed that the trigger signal contains/carries a probability that the catheter is located entirely in the blood pool (i.e. is not in contact with the anatomical structure), which has been derived from a ratio between the first and second measurements as previously described.

The method 700 then comprises a step 720 of determining whether to obtain the calibration data based on the trigger signal. The method moves to step 730 in response to step 720 determining to obtain the calibration data.

Step 720 may, for instance, comprise comparing the probability to some predefined threshold (e.g. for a probability ranging from 0 to 1, which may initially be 0.9), which can be called a "re-zeroing threshold". In response to the probability breaching (e.g. exceeding) this threshold, the method moves to step 730.

Step 730 comprises obtaining calibration data from one or more electrodes of the catheter. The calibration data may, for instance, comprise one or more measurements obtained from the electrodes (e.g. representing a "zero" for the purpose of future measurements).

The calibration data may be used to calibrate future measurements taken by processing circuit 150, e.g. using a commonly known zeroing mechanism. This may take place in a step 735 (which does not need to be performed by the same processing circuitry that carries out the other steps of this method 700). Thus, the trigger signal may effectively control when zeroing (and/or other calibration processes) is/are performed.

Where the trigger signal carries a probability, the method 700 may comprise a first threshold modifying step 740, which is performed (only) when step 730 is performed. The first threshold modifying step 740 may comprise modifying the predefined threshold, e.g. increasing the predefined threshold by a first predetermined amount. The modification is such that (for future iterations of the method) the probability indicator must indicate a greater likelihood that the catheter is located entirely in the blood pool before calibration data is obtained.

After performing step 730 (and/or step 740 if performed), the method reverts back to step 710.

In some examples, and when the trigger signal carries a probability, the method 700 may be configured to modify the predefined threshold over time. For instance, the modification may be configured to gradually (for future interactions) be such that the probability indicator can indicate a lower likelihood that the catheter is located entirely in the blood pool before calibration data is obtained. For instance, the predefined threshold may be reduced by a second predetermined amount.

This process may be performed in optional step 750, which reduces the predefined threshold (e.g. by a predetermined percentage) if a predetermined time period has elapsed since it last reduced the predefined threshold.

Relaxing the predefined threshold in this manner can help accommodate temporal and/or location based changes (e.g. to account for movement of the catheter within the anatomical structure).

In some examples, and when the trigger signal carries a probability, the method 700 may be configured to modify the predefined threshold responsive to a movement of the catheter.

This may be performed in optional step 760, which obtains movement data and reduces the predefined threshold if a movement beyond some predetermined threshold has elapsed since it last reduced the predefined threshold. The movement data may comprise any data responsive to a movement (e.g. a change in location based on determined locations of the catheter and/or movement data derived from an accelerometer positioned on the catheter).

In some examples, all of the steps of method 700 (excluding step 730) may be performed during the generation of the trigger signal (e.g. in step 250). In this scenario, the trigger signal may comprise a binary indicator (e.g. a "0" or "1") that indicates whether or not the calibration data is to be obtained. Thus, the processor circuit may effectively perform, as part of step 250, method 700 (excluding step 730), with step 730 being performed by other processing circuitry - e.g. responsive to the binary indicator.

Of course, in some examples, the processor circuit 140 and the processing circuity 150 are integrated into a same processing arrangement.

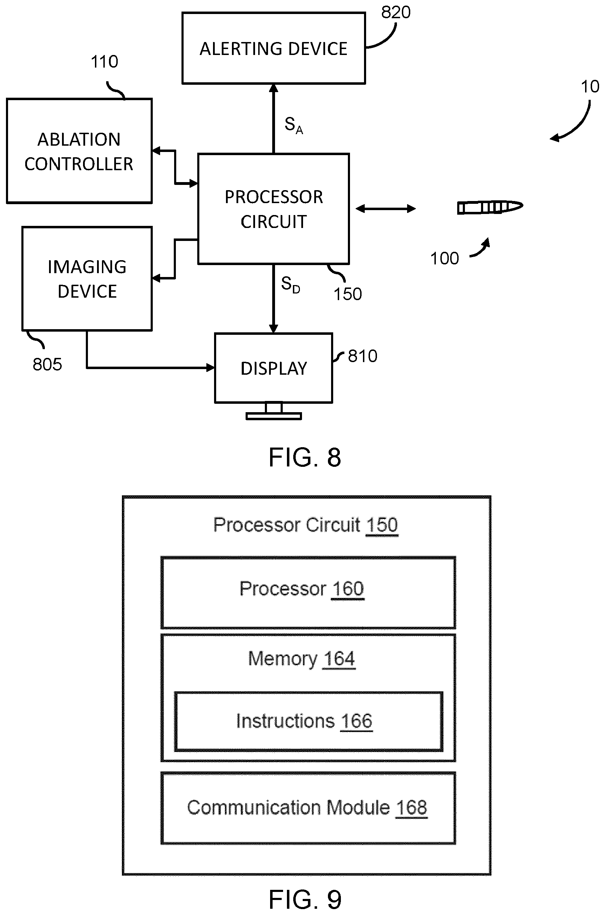

Fig. 8 illustrates some further optional elements for the ablation system 10.

The ablation system of Fig. 8 may be modified to provide other types of catheter systems (e.g. employing other types of catheter). For instance, the ablation therapy catheter 100 may be replaced by another type of catheter (such as those previously described), and the ablation controller 110 may be omitted. Reference to an "ablation system" may be replaced by the term "catheter system" where appropriate.

The ablation system may comprise an imaging system 805 configured to generate an image of the anatomical structure. The imaging system may operate, for example, by monitoring the position of the catheter with respect to a three-dimensional space and generating an anatomical model of the anatomical structure based on the positions of the catheter. Approaches for generating an anatomical model in this manner will be apparent to the skilled person, e.g. using previously identified documents U.S. Patent No. 10,278,616 and U.S. Patent No. 5,983,126 or from Romanov, Alexander, et al. "High-resolution, real-time, and nonfluoroscopic 3-dimensional cardiac imaging and catheter navigation in humans using a novel dielectric-based system." Heart rhythm 16.12 (2019): 1883-1889.

The imaging system may control a display to generate a visual representation of the anatomical model, and optionally the position of the catheter with respect to the anatomical model.

The prediction signal S_{D} generated using the present invention may be used to improve the accuracy of the anatomical model. In particular, contact between the catheter and the anatomical structure may result in the catheter deforming the anatomical structure. This would lead to the imaging system inaccurately determining a shape of the anatomical structure (e.g. compared to a non-deformed anatomical structure).

The prediction signal may therefore be used by the imaging system to disregard data (for generating the anatomical model) when the catheter is predicted to be touching the anatomical model (with more than a threshold amount of force), e.g. when too high a force is applied by the catheter. This increases an accuracy of the anatomical model, and therefore of a clinicians understanding of the state of the anatomical structure.

In some examples, a visual representation of the anatomical model may be modified to indicate a location of a touch, e.g. by tracking the position of the catheter and detecting when a touch occurs.

Information on whether or not the catheter is touching the anatomical structure can be used to improve the generation of the anatomical model of the anatomical structure. For example, positions of the catheter that represent positions of contact with the anatomical structure (e.g. obtained using the proposed method) could be weighted more than positions of the catheter that do not represent positions of contact with the anatomical structure. This approach can improve the generation of the anatomical model, as positions of the catheter when it (just) makes contact with the anatomical structure mark the true boundaries of the anatomical structure. This concept for improving the generation of an anatomical model using contact information could form a separate inventive concept.

Just as contact information (e.g. the predictive signal) can be used to improve the accuracy of a mapping, some embodiments may use mapping to improve contact detection.

The ablation system may comprise an ablation controller 110, previously described. The ablation controller 110 is configured to control the ablation performed by ablation electrodes of the catheter 100.

The ablation controller may be configured to (automatically) control the ablation responsive to an indication of whether or not contact is made between the catheter 100 and the anatomical structure (from the predictive signal S_{P}). In particular, the ablation controller may be configured to control an ablation current provided to the ablation electrodes of the catheter 100 responsive to the predictive signal S_{P}.

For example, the ablation controller may be configured to provide ablation only when contact is made between the catheter and the anatomical structure (and is in the correct position). As another example, the ablation controller may be configured to control a magnitude of ablation responsive to a contact force between the catheter and the anatomical structure.

It is recognized that, in order to ablate efficiently, the catheter should not touch the tissue either too lightly or firmly. For example, if the catheter touches the tissue too lightly, then ablation will be efficient, whereas if the catheter touches the tissue too firmly it can create a hole during the ablation.

Other examples of (automatically) controlling the ablation based on a prediction of whether or not contact is made between the catheter and the anatomical structure will be apparent to the skilled person.

The ablation system 10 may comprise a display or user interface 810.

The processor circuit may be configured to generate a display signal S_{D} responsive to the prediction of whether or not the catheter is in contact with the anatomical structure (e.g. the predicted contact force).

The display 810 may be configured to receive the display signal S_{D} and control a visual output of the display based on the display signal. In this way, a clinician or operator can be provided with an indication of whether or not contact has been made and optionally a level of the predicted contact force.

The display signal may, for example, directly control a visual output of the display (e.g. carry display data) of the prediction of whether or not the catheter is in contact with the anatomical structure (and optionally a predicted contact force), or indirectly control (e.g. by carrying data which is subsequently interpreted by the display or user interface).

The display signal may comprise any suitable type of communication, such as an electrical signal (e.g., digital electrical signal) representative of image data or other data for influencing the display. For example, the electrical signal may be in a format suitable for display by a display (such as a computer monitor, television screen, mobile computing device display, etc.).

In some examples, the display 810 may be adapted to provide the visual representation of the anatomical model generated by the imaging system 805 (if present). The visual representation may also display a relative location of the catheter with respect to the anatomical model. The display 810 may be adapted to modify the visual representation of the anatomical model based on the display signal S_{D}, e.g. to indicate a location of a touch.

The ablation system 10 may comprise a user-perceptible alerting device 820 (e.g. an alarm or buzzer). The user-perceptible alerting device may be configured to provide a user-perceptible output, e.g. an audio, visual or haptic output.

The processor circuit may be configured to generate an alert signal S_{A} that controls the user-perceptible output provided by user-perceptible alerting device responsive to the prediction of whether or not the catheter is in contact with the anatomical structure. One or more characteristics of the user-perceptible output may be adjusted responsive to a detected contact force.

For example, where the user-perceptible alerting device is adapted to provide an audio output, the processor circuit may be configured to cause the alerting device to generate a tone in response to detecting that the catheter is in contact with the anatomical structure (and no tone if no contact is made). In some examples, one or more characteristics of this tone (e.g. volume, frequency, pattern etc.) changes as a contact force increases (e.g. increases in pitch).

This signal may, for example, be provided to a display or user interface that generates a display responsive to the generated signal. In some examples, the signal is provided to a storage, for storing generated indicators (e.g. for later processing or review). In yet other examples, the signal is provided to an alarm module that, responsive to the signal meeting some predetermined criteria, generates a user-perceptible alarm. Other uses and purposes for the signal will be apparent to the skilled person.

The signal may, for example, directly control a visual output of the display (e.g. carry display data) of the two or more indicators and/or additional information derived therefrom, or indirectly control (e.g. by carrying data which is subsequently interpreted by the display or user interface).

In some examples, the signal may comprise any suitable type of communication, such as an electrical signal (e.g., digital electrical signal) representative of image data or other data for influencing the display. For example, the electrical signal may be in a format suitable for display by a display device (e.g., computer monitor, television screen, mobile computing device display, etc.).

The electrodes used in the present disclosure may be repurposed electrodes already existing on/in the catheter, such as ablation electrodes or electrodes used to map the anatomical structure and/or locate the catheter. Thus, the present disclosure has a further advantage in not requiring additional electrodes to be mounted on the catheter.

Fig. 9 is a schematic diagram of a processor circuit 150, according to embodiments of the present disclosure. As shown, the processor circuit 150 may include a data processor 160, a memory 164, and a communication module 168. These elements may be in direct or indirect communication with each other, for example via one or more buses.

The processor circuit 150 may be formed as a single device or may be distributed across a plurality of devices (e.g. a cloud-computing network).

The data processor 160 may include a central processing unit (CPU), a digital signal processor (DSP), an ASIC, a controller, an FPGA, another hardware device, a firmware device, or any combination thereof configured to perform the operations described herein. The data processor160 may also be implemented as a combination of computing devices, e.g., a combination of a DSP and a microprocessor, a plurality of microprocessors, one or more microprocessors in conjunction with a DSP core, or any other such configuration. In some embodiments, the processor is a distributed processing system, e.g. formed of a set of distributed processors.

The memory 164 may include a cache memory (e.g., a cache memory of the data processor160), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, other forms of volatile and non-volatile memory, or a combination of different types of memory. In an embodiment, the memory 164 includes a non-transitory computer-readable medium. The non-transitory computer-readable medium may store instructions. For example, the memory 164, or non-transitory computer-readable medium may have program code recorded thereon, the program code including instructions for causing the processor circuit 150, or one or more components of the processor circuit 150, to perform the operations described herein. For example, the processor circuit 150 can execute operations of the methods 200, 700. Instructions 166 may also be referred to as code or program code. The terms "instructions" and "code" should be interpreted broadly to include any type of computer-readable statement(s). For example, the terms "instructions" and "code" may refer to one or more programs, routines, sub-routines, functions, procedures, etc. "Instructions" and "code" may include a single computer-readable statement or many computer-readable statements. The memory 164, with the code recorded thereon, may be referred to as a computer program product.

The communication module 168 can include any electronic circuitry and/or logic circuitry to facilitate direct or indirect communication of data between the processor circuit 150, the mapping and guidance system 114, the catheter 100, the cryoballoon catheter 130, and/or the display 510. In that regard, the communication module 168 can be an input/output (I/O) device. In some instances, the communication module 168 facilitates direct or indirect communication between various elements of the processor circuit 150 and/or the system 10 (Fig. 1).

Methods of tracking the position of a catheter and generating an anatomical model of an anatomical structure from the positions of the catheter have been described in this disclosure. Once an anatomical model of an anatomical structure is available, subsequent positions of the catheter with respect to the anatomical model/structure can be tracked, e.g. by monitoring the position of the catheter with respect to the same 3D space from which the anatomical model was generated.

This information can be processed to predict a distance (a "model-derived distance") between the catheter and the anatomical structure. This distance is sometimes called the distance-to-mesh (i.e. the distance between the catheter and the anatomical model - which is usually a mesh). It will be apparent that the model-derived distance will change as the catheter is moved with respect to the anatomical structure.

The model-derived distance can be used to predict whether or not contact is made between the catheter and the anatomical structure. For example, where the model-derived distance is 0, the model predicts that the catheter is in contact with the anatomical structure. Negative values of model-derived distances may represent a magnitude of contact force between the catheter and the anatomical structure, whereas positive values may represent a gap between the catheter and the anatomical structure. This model-derived prediction of contact is largely independent of the occurrence of ablation.

In particular examples, touch information responsive to a touch between the catheter and the anatomical structure can be used to calibrate a model-derived distance. The predictive signal proposed by previously described embodiments carries touch information according to one example of this concept. In particular, the touch information may be generated independently of the anatomical model (e.g. by directly processing electrical response(s) of electrode(s) of a catheter without generating an anatomical model).

Once the touch information indicates a contact is made between the catheter and the anatomical structure (e.g. using herein described algorithmic processes that use catheter electrodes), the model-derived distance at this location can be recorded. In particular, the model-derived distance can be recorded the first time that the touch information indicates that the catheter makes contact with the anatomical structure. This recorded model-derived distance provides a calibration or reference distance.

The difference between this "recorded model-derived distance" and a subsequently determined model-derived distance (e.g. an "ongoing model-derived distance") can more accurately represent a magnitude of touch or contact force (or distance) between the catheter and the anatomical structure.

In particular, if there is no change in the ongoing model-derived distance from the recorded model-derived distance, then the catheter makes a same level of contact with the anatomical structure. If the recorded distance is greater than the ongoing distance, then the catheter is applying a greater contact force to the anatomical structure. If the recorded distance is less than the ongoing distance, then the catheter is moving away from the anatomical structure (i.e. a gap is forming).

Thus, a difference between the recorded distance and the ongoing distance can indicate a magnitude of contact force and/or a distance between the catheter and the anatomical structure.

The difference between the recorded distance and the ongoing distance may be compared to one or more thresholds to identify one or more predicted touch levels (e.g. no touch, high touch, very high touch) between the catheter and the anatomical structure. Thus, additional contact information and/or context can be derived from the difference.

It is also recognized that if the recorded model-derived distance is too large or too small (e.g. too far below zero), it can be predicted that the mapping or creation of the anatomical model was inaccurate. Thus, some embodiments may comprise a step of comparing the recorded model-derived distance to one or more predetermined thresholds and, if the recorded model-derived distance breaches the threshold(s), generating a model accuracy alert. The model accuracy alert may cause a model generation (for generating the anatomical model) to be repeated.

The preceding passages provide an approach for determining or generating a "distance-to-mesh" between an electrode and the bounds of an anatomical cavity. This distance-to-mesh may be advantageously used in other herein-described methods.

It will be understood that disclosed methods are preferably computer-implemented methods. As such, there is also proposed the concept of a computer program comprising computer program code for implementing any described method when said program is run on a processing system, such as a computer or a set of distributed processors.

Different portions, lines or blocks of code of a computer program according to an embodiment may be executed by a processing system or computer to perform any herein described method. In some alternative implementations, the functions noted in the block diagram(s) or flow chart(s) may occur out of the order noted in the figures. For example, two blocks shown in succession may, in fact, be executed substantially concurrently, or the blocks may sometimes be executed in the reverse order, depending upon the functionality involved.

The present disclosure proposes a computer program (product) comprising instructions which, when the program is executed by a computer or processing system, cause the computer or processing system to carry out (the steps of) any herein described method. The computer program (product) may be stored on a non-transitory computer readable medium.

Similarly, there is also proposed a computer-readable (storage) medium comprising instructions which, when executed by a computer or processing system, cause the computer or processing system to carry out (the steps of) any herein described method. There is also proposed computer-readable data carrier having stored thereon the computer program (product) previously described. There is also proposed a data carrier signal carrying the computer program (product) previously described.

The computer-readable program may execute entirely on a single computer/processor, partly on the computer/processor, as a stand-alone software package, partly on the computer/processor and partly on a remote computer or entirely on the remote computer or server (e.g. using a distributed processor processing system). In the latter scenario, the remote computer may be connected to the computer/processor through any type of network, including a local area network (LAN) or a wide area network (WAN), or the connection may be made to an external computer (for example, through the Internet using an Internet Service Provider).

It will also be understood that the embodiments described above are exemplary and are not intended to limit the scope of the disclosure to a given clinical application. For example the devices, systems, and techniques described above can be used in a variety of ablation applications that involve the ablation of feature of an anatomical structure. The techniques described above can be used to guide an RF ablation procedure in which one or more RF ablation electrodes are used to create an electrically-isolating lesion in cardiac tissue. Of course, the techniques described above could also be used to guide any other suitable ablation procedure, such as a cryo-ablation or pulsed field ablation procedure.

Further, while the embodiments are described with respect to the heart and associated anatomy, it will be understood that the same methods and systems can be used for procedures in other body volumes, including other regions of interest in the heart, or other body cavities and/or lumens. For example, in some embodiments, the procedures described herein can be used alongside treatment, surgical or investigative procedures in any number of anatomical locations and tissue types, including without limitation, organs including the liver, heart, kidneys, gall bladder, pancreas, lungs; ducts; intestines; nervous system structures including the brain, dural sac, spinal cord and peripheral nerves; the urinary tract; as well as valves within the blood, chambers or other parts of the heart, and/or other systems of the body. The anatomy may be a blood vessel, as an artery or a vein of a patient's vascular system, including cardiac vasculature, peripheral vasculature, neural vasculature, renal vasculature, and/or any other suitable lumen inside the body. In addition to natural structures, the approaches described herein may be used to examine man-made structures such as, but without limitation, heart valves, stents, shunts, filters and other devices in the kidneys, lungs, or any other suitable body volume. Thus, the anatomical structure referred to in the present disclosure may include any suitable organ (such as those previously listed), blood vessel and/or man-made structures.

Persons skilled in the art will recognize that the apparatus, systems, and methods described above can be modified in various ways. Accordingly, persons of ordinary skill in the art will appreciate that the embodiments encompassed by the present disclosure are not limited to the particular exemplary embodiments described above. In that regard, although illustrative embodiments have been shown and described, a wide range of modification, change, and substitution is contemplated in the foregoing disclosure. It is understood that such variations may be made to the foregoing without departing from the scope of the present disclosure. Accordingly, it is appropriate that the appended claims be construed broadly and in a manner consistent with the present disclosure.

## Claims

1. A processor circuit (150) for generating and outputting a trigger signal for controlling the collection of electrode calibration data from a catheter, comprising two or more electrodes, for insertion into one or more cavities of an anatomical structure, the processor circuit being configured for communication with the catheter, the processor circuit comprising:
an input interface (151) configured to obtain (210, 220):
a first measurement (310, 410), from a first set of one or more electrodes of the catheter, responsive to a change in contact and/or distance between the first set of two or more electrodes and the anatomical structure; and
a second measurement (320, 420), from a second, different set of one or more electrodes of the catheter, responsive to a change in contact and/or distance between the second set of two or more electrodes and the anatomical structure, and
a data processor communicatively coupled to the input interface and configured to:
determine (230) a ratio (330, 430) between the first and second measurements; and
process (240) the determined ratio to generate a predictive indicator that indicates a likelihood that the ablation therapy catheter is in contact with the anatomical structure; and
generate (250) and output a trigger signal that changes responsive to the predictive indicator.

2. The processor circuit of claim 1, wherein the catheter is an ablation therapy catheter, and the first set of one or more electrodes of the ablation therapy catheter (100) comprise one or more electrodes (101) used to apply an ablation treatment to the anatomical structure.

3. The processor circuit of any of claims 1 or 2, wherein the first measurement comprises an impedance measurement between a first set of two electrodes of the catheter.

4. The processor circuit of claim 3, wherein the second measurement comprises an impedance measurement between a second set of two electrodes of the catheter, wherein the second set is different to the first set.

5. The processor circuit of any of claims 1 to 4, wherein the data processor is configured to process the determined ratio by processing the determined ratio and the first measurement to generate the predictive indicator.

6. The processor circuit of any of claims 1 to 5, wherein:
the input interface is configured to receive a physiological signal, the physiological signal responsive to a respiratory and/or cardiac movement of the patient; and
the data processor is configured to process the determined ratio by processing the determined ratio and the physiological signal to generate the predictive indicator.

7. The processor circuit of any of claims 1 to 6, wherein the data processor is configured to process the determined ratio by comparing the determined ratio to one or more first predetermined values and/or ranges to generate a predictive indicator that predicts whether or not the ablation therapy catheter is in contact with the anatomical structure.

8. The processor circuit of any of claims 1 to 6, wherein the data processor is configured to:
process the determined ratio by processing the determined ratio using a probability function to calculate a probability that the ablation therapy catheter is in contact with the anatomical structure; and
generate the trigger signal responsive to the calculated probability.

9. The processor circuit of claim 8, wherein the probability function defines a predetermined mapping between possible values for the determined ratio and possible values for the probability.

10. The processor circuit of claim 8 or 9, wherein the data processor is configured to generate the trigger signal to carry the calculated probability.

11. A processing arrangement comprising:
the processor circuit of any of claims 1 to 10; and
processing circuitry configured to:
receive (710) the trigger signal output by the processor circuit; and
selectively obtain (720, 730) electrode calibration data, from one or more of the electrodes of the catheter, responsive to the trigger signal.

12. The processing arrangement of claim 11, wherein:
the processor circuit is the processor circuit of claim 10; and
the processing circuitry is configured to obtain the electrode calibration data in response to the calculated probability, contained in the trigger signal, exceeding a predetermined probability threshold.

13. A computer-implemented method (200) for generating and outputting a trigger signal for controlling the collection of electrode calibration data using an ablation therapy catheter, comprising two or more electrodes (101, 102, 103, 104), for insertion into one or more cavities of an anatomical structure, the method comprising:
obtaining (210) a first measurement (310, 410), from a first set of one or more electrodes of the catheter, responsive to a change in contact and/or distance between the first set of two or more electrodes and the anatomical structure;
obtaining (220) a second measurement (320, 420), from a second set of one or more electrodes of the catheter, responsive to a change in contact and/or distance between the second set of two or more electrodes and the anatomical structure;
determining (230) a ratio (330, 430) between the first and second measurements;
processing (240) the determined ratio to generate a predictive indicator that indicates a likelihood that the ablation therapy catheter is in contact with the anatomical structure; and
generating (250) and outputting a trigger signal that changes responsive to the predictive indicator.

14. A computer program product comprising code which, when executed by a processor circuit, causes the processor circuit to perform the steps of the method according to claim 13.

15. A non-transitory computer-readable medium or data carrier comprising or carrying the computer program product of claim 14.
